# EUROPEAN PATENT SPECIFICATION

(11) **EP 0 652 004 B1**
(45) Date of publication and mention of the grant of the patent: **10.07.2002**
(21) Application number: 94307527.5
(22) Date of filing: 13.10.1994
(51) Int. Cl.: A61K 31/4465, A61P 35/00

(54) **Methods for treating resistant neoplasms**
Methode zur Behandlung von resistenten Neoplasmen
Méthode pour le traitement de néoplasmes résistants

(30) Priority: 15.10.1993 US 137678
(43) Date of publication of application: 10.05.1995
(73) Proprietor: ELI LILLY AND COMPANY, Indianapolis, Indiana 46285 (US)
(72) Inventor: Chandrasekhar, Srinivasan, Indianapolis, Indiana 46256 (US); Dantzig, Anne Hollins, Crawfordsville, Indiana 47933 (US); Shepard, Robert Lee, Nobelsville, Indiana 46060 (US); Starling, James Jacob, Carmel, Indiana 46032 (US); Winter, Mark Alan, Indianapolis, Indiana 46220 (US)
(74) Representative: Vaughan, Jennifer Ann

(56) References cited:
- US-A- 4 133 814
- US-A- 4 418 068
- J. CLIN. ONCOL., vol. 7, no.6, 1989 pages 710-717, K. OSBORNE ET AL. 'Antagonism of chemotherapy-induced cytotoxicity for human brest cancer cells by antiestrogens'
- INT. J. CANCER, vol. 45, no.4, 1990 pages 712-718, NG. COLDHAM ET AL. 'Hormone and cytotoxic drug responsiveness of cultured human breast cancer cells resistant to specific hormones'
- CANCER RES., vol. 49, no.19, 1989 pages 5312-5316, DT. KIANG ET AL. 'Up-regulation of estrogen receptors by nonsteroidal antiestrogens in human breast cancer'

## Description

Along with surgery and radiotherapy, chemotherapy continues to be an effective therapy for many cancers. In fact, several types of cancer are now considered to be curable by chemotherapy and include Hodgkin's disease, large cell lymphoma, acute lymphocytic leukemia, testicular cancer and early stage breast cancer. Other cancers such as ovarian cancer, small cell lung and advanced breast cancer, while not yet curable, are exhibiting positive response to combination chemotherapy. One of the most important unsolved problems in cancer treatment is drug resistance. Drug resistance includes both intrinsic resistance at the time of treatment using chemotherapy and acquired drug resistance. This problem is a reason for the added importance of combination chemotherapy, as the therapy both has to avoid the emergence of resistant cells and to kill pre-existing cells which are already drug resistant.

Anthracyclines represent an important class of oncolytic agents. Doxorubicin, an anthracycline, which is also known in the art as Adriamycin™, is a drug of choice in the clinical management of breast cancer. Therapy with anthracyclines such as doxorubicin is complicated by the appearance of the anthracycline resistant phenotype which limits or negates the oncolytic activity of doxorubicin.

Topoisomerase inhibitors represent a further class of oncolytic agents. Epipodophyllotoxins such as Etoposide® and Teniposide® are topoisomerase inhibitors which are useful in the therapy of neoplasms of the testis, small-cell lung and other lung, breast, Hodgkin's disease, non-Hodgkin's lymphomas, acute granulocytic leukemia and Karposi's sarcoma. The therapeutic utility of the epipodophylotoxins is limited by the appearance of the epipodophyllotoxin resistant phenotype.

One form of multi-drug resistance, (MDR), is mediated by a membrane bound 170-180 kD energy-dependent efflux pump designated as P-glycoProtein, P-gp. P-gp has been shown to play a major role in the intrinsic and acquired resistance of a number of human tumors against hydrophobic, natural product drugs. Drugs that act as substrates for and are consequently detoxified by P-gp include the vinca alkaloids (vincristine and vinblastine), anthracyclines (Adriamycin), and epipodophyllotoxins (etoposide). While P-gp associated MDR is a major determinant in tumor cell resistance to chemotherapeutic agents, it is clear that the phenomenon of MDR is multifactorial and involves a number of different mechanisms. One such alternative pathway for resistance to anthracyclines involves the emergence of a 190 kD protein that is not P-gp. *See* McGrath, T., Latoud, C., Arnold, S.T., Safa, A.R., Felsted, R.S., and Center, M.S. Biochem. Pharmacol., **38:** 3611, (1989). P190 is not found on the plasma membrane but rather appears to be localized predominantly in the endoplasmic reticulum *See* Marquardt, D. and Center, M.S. Cancer Res., **52:** 3157, (1992).

P190 possesses a nucleotide binding domain that is homologous with the ATP binding site of P-gp *See* Marquardt, D., McCrone, S., and Center M.S., Cancer Res., **50:** 1426, (1990). The mechanism(s) utilized by P190 to confer resistance to Adriamycin is not well understood but may involve the intracellular redistribution of Adriamycin away from the nucleus. *See* Marquardt, D. and Center, M.S.,supra. Adriamycin is an inhibitor of topoisomerase II (Beck, W.T., Bull. Cancer, **77:** 1131, (1990), which is an enzyme involved in DNA replication. Redistribution of Adriamycin away from the nucleus would therefore be an important component in cellular resistance to this drug. The studies published to date on P190 have utilized cell lines selected *in vitro* for resistance to Adriamycin (McGrath, T., Latoud, C., Arnold, S.T., Safa, A.R., Felsted, R.S., and Center, M.S., *supra;* Marquardt, D. and Center, M.S., *supra;* and Marquardt, D., McCrone, S., and Center M.S. Cancer Res., *supra.* The association of P190 with drug resistance was made by sodium dodecyl sulfate polyacrylamide gel electrophoresis (SDS-PAGE) of radioactive extracts prepared from Adriamycin-resistant HL60/Adr human leukemia cells labeled with 8-azido-alpha[³²P]ATP. *See* McGrath, T., Latoud, C., Arnold, S.T., Safa, A.R., Felsted, R.S., and Center, M.S., *supra.* The drug-resistance phenotype conferred by P190 is not limited to the anthracyclines. Epipodophyllotoxin resistance is linked to P190 expression. The IC₅₀s of HL60/S cells treated with Adriamycin and Etoposide were 0.011 µg/ml and 0.39 µg/ml respectively. The IC₅₀s for HL60/Adr cells (a HL60-derived cell line which is resistant to doxorubicin) treated with Adriamycin and Etoposide were 2.2 µg/ml and >10 µg/ml respectively. HL60/S and HL60/Adr cell lines do not express P-glycoProtein. HL60/Adr expresses P190. Thus, resistance to the anthracyclines and epipodophyllotoxins results from P190 expression.

Therefore, it would be desirable to provide compounds which are useful for treating resistant neoplasms, the resistant pathway including P190, p-glycoProtein, or both. N. G Goldham et al., Int. J. Cancer, 1990, 45, 712 describes the use of certain benzothiophene derivatives to improve anti-oestrogen resistance in MCF-7 cells. C. Osborne et al., Journal of Clinical Oncology, 1989,7,710 describes the antagonistic interaction of tamoxifen with cytotoxic drugs in human breast cancer cell lines.

This invention provides the use of a compound of formula I

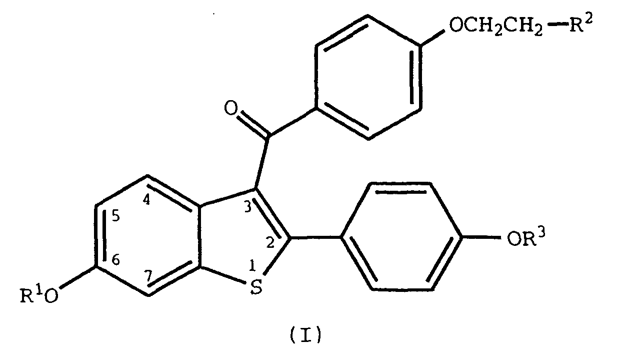

Wherein R1 and R3 are each hydrogen; R² is piperidino; and pharmaceutically acceptable salts and solvates thereof, in the preparation of a medicament for reversing drug resistance in a drug-resistant neoplasm.

The current invention concerns the discovery that a select group of 2-phenyl-3-aroylbenzothiophenes (benzothiophenes), those of formula I, are useful for treating resistant neoplasms. The medicaments provided by this invention are used by administering to a human or other mammal in need a dose of a compound of formula I or a pharmaceutically acceptable salt or solvate thereof, that is effective to make the neoplasms less resistant to chemotherapy. In making the neoplasm less resistant, the compounds of the invention may be used on neoplasms having intrinsic and/or acquired resistance. Such neoplasms include those which have a pathway for resistance which includes the protein P190. Resistance to drugs such as epipodophyllotoxins and anthracyclines are linked to P190. The treatment of the resistant and succeptible neoplasm will result in a reversal or inhibition of resistance, or in other words, will cause the neoplasm to be more sensitive to the appropriate chemotherapy.

The compounds of the invention may be used for many resistant neoplasms, including colon cancer, mesothelioma, melanoma, prostate cancer, ovarian cancer, non-small cell lung cancer, small-cell lung cancer, bladder cancer, endometrial cancer, leukemia, testicular cancer, breast cancer, and large cell lymphoma. More particular types of cancer are Hodgkin's disease, Karposi's sarcoma, and acute granulocytic leukemia.

Generally, the compound is formulated with common excipients, diluents or carriers, and compressed into tablets, or formulated as elixirs or solutions for convenient oral administration, or administered by the intramuscular or intravenous routes. The compounds can be administered transdermally, and may be formulated as sustained release dosage forms and the like.

The compounds used in the methods of the current invention can be made according to established procedures, such as those detailed in U.S. Patent Nos. 4,133,814, 4,418,068, and 4,380,635, all of which are incorporated by reference herein. In general, the process starts with a benzo[b]thiophene having a 6-hydroxyl group and a 2-(4-hydroxyphenyl) group. The starting compound is protected, acylated, and deprotected to form the formula I compounds. Examples of the preparation of such compounds are provided in the U.S. patents discussed above. Substituted phenyl includes phenyl substituted once or twice with C₁-C₆ alkyl, C₁-C₄ alkoxy, hydroxy, nitro, chloro, fluoro, or tri(chloro or fluoro)methyl.

The compounds used in the methods of this invention form pharmaceutically acceptable acid and base addition salts with a wide variety of organic and inorganic acids and bases and include the physiologically acceptable salts which are often used in pharmaceutical chemistry. Such salts are also part of this invention. Typical inorganic acids used to form such salts include hydrochloric, hydrobromic, hydroiodic, nitric, sulfuric, phosphoric, hypophosphoric and the like. Salts derived from organic acids, such as aliphatic mono and dicarboxylic acids, phenyl substituted alkanoic acids, hydroxyalkanoic and hydroxyalkandioic acids, aromatic acids, aliphatic and aromatic sulfonic acids, may also be used. Such pharmaceutically acceptable salts thus include acetate, phenylacetate, trifluoroacetate, acrylate, ascorbate, benzoate, chlorobenzoate, dinitrobenzoate, hydroxybenzoate, methoxybenzoate, methylbenzoate, o-acetoxybenzoate, naphthalene-2-benzoate, bromide, isobutyrate, phenylbutyrate, β-hydroxybutyrate, butyne-1,4-dioate, hexyne-1,4-dioate, caprate, caprylate, chloride, cinnamate, citrate, formate, fumarate, glycollate, heptanoate, hippurate, lactate, malate, maleate, hydroxymaleate, malonate, mandelate, mesylate, nicotinate, isonicotinate, nitrate, oxalate, phthalate, teraphthalate, phosphate, monohydrogenphosphate, dihydrogenphosphate, metaphosphate, pyrophosphate, propiolate, propionate, phenylpropionate, salicylate, sebacate, succinate, suberate, sulfate, bisulfate, pyrosulfate, sulfite, bisulfite, sulfonate, benzene-sulfonate, p-bromophenylsulfonate, chlorobenzenesulfonate, ethanesulfonate, 2-hydroxyethanesulfonate, methane-sulfonate, naphthalene-1-sulfonate, naphthalene-2-sulfonate, p-toluenesulfonate, xylenesulfonate, tartarate, and the like. A preferable salt is the hydrochloride salt.

The pharmaceutically acceptable acid addition salts are typically formed by reacting a compound of formula I with an equimolar or excess amount of acid. The reactants are generally combined in a mutual solvent such as diethyl ether or benzene. The salt normally precipitates out of solution within about one hour to 10 days and can be isolated by filtration or the solvent can be stripped off by conventional means.

Bases commonly used for formation of salts include ammonium hydroxide and alkali and alkaline earth metal hydroxides, carbonates and bicarbonates, as well as aliphatic and aromatic amines, aliphatic diamines and hydroxy alkylamines. Bases especially useful in the preparation of addition salts include ammonium hydroxide, potassium carbonate, sodium bicarbonate, calcium hydroxide, methylamine, diethylamine, ethylene diamine, cyclohexylamine and ethanolamine.

The pharmaceutically acceptable salts generally have enhanced solubility characteristics compared to the compound from which they are derived, and thus are often more amenable to formulation as liquids or emulsions.

Pharmaceutical formulations can be prepared by procedures known in the art. For example, the compounds can be formulated with common excipients, diluents, or carriers, and formed into tablets, capsules, suspensions, powders, and the like. Examples of excipients, diluents, and carriers that are suitable for such formulations include the following: fillers and extenders such as starch, sugars, mannitol, and silicic derivatives; binding agents such as carboxymethyl cellulose and other cellulose derivatives, alginates, gelatin, and polyvinyl pyrrolidone; moisturizing agents such as glycerol; disintegrating agents such as agaragar, calcium carbonate, and sodium bicarbonate; agents for retarding dissolution such as paraffin; resorption accelerators such as quaternary ammonium compounds; surface active agents such as acetyl alcohol, glycerol monostearate; adsorptive carriers such as kaolin and bentonite; and lubricants such as talc, calcium and magnesium stearate, and solid polyethyl glycols.

The compounds can also be formulated as elixirs or solutions for convenient oral administration or as solutions appropriate for parenteral administration, for instance by intramuscular, subcutaneous or intravenous routes. Additionally, the compounds are well suited to formulation as sustained release dosage forms and the like. The formulations can be so constituted that they release the active ingredient only or preferably in a particular part of the intestinal tract, possibly over a period of time. The coatings, envelopes, and protective matrices may be made, for example, from polymeric substances or waxes.

The particular dosage of a compound of formula I required to treat resistant neoplasms, according to this invention, will depend upon the severity of the condition, the route of administration, and related factors that will be decided by the attending physician. Generally, accepted and effective daily doses will be from 0.1 to 2000 mg/day, although the attending physician may increase the dose, based upon the factors above. Such dosages will be administered to a subject in need of treatment from once to about three times each day, or more often as needed to effectively treat resistant neoplasms.

It is usually preferred to administer a compound of formula I in the form of an acid addition salt, as is customary in the administration of pharmaceuticals bearing a basic group, such as the piperidino ring. It is also advantageous to administer such a compound by the oral route. For such purposes the following oral dosage forms are available.

Another aspect of the invention is the combination treatment using the compounds of the invention and a chemotherapeutic agent or agents. Such combination treatment includes treatment first with a compound of the invention followed by an agent or agents, simultaneous use of a compound of the invention and an agent or agents, or use of a compound of the invention after treatment with an agent or agents.

### Formulations

In the formulations which follow, "Active ingredient" means a compound of formula I.

### Formulation 1: Gelatin Capsules

Hard gelatin capsules are prepared using the following:

| Ingredient | Quantity (mg/capsule) |
|---|---|
| Active ingredient | 0.1 - 2000 |
| Starch, NF | 0 - 650 |
| Starch flowable powder | 0 - 650 |
| Silicone fluid 350 centistokes | 0 - 15 |

The ingredients are blended, passed through a No. 45 mesh U.S. sieve, and filled into hard gelatin capsules.

Examples of specific capsule formulations of the compound of formula 1 wherein R² is 1-piperidino, (raloxifene), that have been made include those shown below:

### Formulation 2: Raloxifene capsule

| Ingredient | Quantity (mg/capsule) |
|---|---|
| Raloxifene | 1 |
| Starch, NF | 112 |
| Starch flowable powder | 225.3 |
| Silicone fluid 350 centistokes | 1.7 |

### Formulation 3: Raloxifene capsule

| Ingredient | Quantity (mg/capsule) |
|---|---|
| Raloxifene | 5 |
| Starch, NF | 108 |
| Starch flowable powder | 225.3 |
| Silicone fluid 350 centistokes | 1.7 |

### Formulation 4: Raloxifene capsule

| Ingredient | Quantity (mg/capsule) |
|---|---|
| Raloxifene | 10 |
| Starch, NF | 103 |
| Starch flowable powder | 225.3 |
| Silicone fluid 350 centistokes | 1.7 |

### Formulation 5: Raloxifene capsule

| Ingredient | Quantity (mg/capsule) |
|---|---|
| Raloxifene | 50 |
| Starch, NF | 150 |
| Starch flowable powder | 397 |
| Silicone fluid 350 centistokes | 3.0 |

The specific formulations above may be changed in compliance with the reasonable variations provided.

A tablet formulation is prepared using the ingredients below:

### Formulation 6: Tablets

| Ingredient | Quantity (mg/tablet) |
|---|---|
| Active ingredient | 0.1 - 2000 |
| Cellulose, microcrystalline | 0 - 650 |
| Silicone dioxide, fumed | 0 - 650 |
| Stearate acid | 0 - 15 |

The components are blended and compressed to form tablets.

Alternatively, tablets each containing 0.1 - 2000 mg of active ingredient are made up as follows:

### Formulation 7: Tablets

| Ingredient | Quantity (mg/tablet) |
|---|---|
| Active ingredient | 0.1 - 2000 |
| Starch | 45 |
| Cellulose, microcrystalline | 35 |
| Polyvinylpyrrolidone (as 10% solution in water) | 4 |
| Sodium carboxymethyl cellulose | 4.5 |
| Magnesium stearate | 0.5 |
| Talc | 1 |

The active ingredient, starch, and cellulose are passed through a No. 45 mesh U.S. sieve and mixed thoroughly. The solution of polyvinylpyrrolidone is mixed with the resultant powders which are then passed through a No. 14 mesh U.S. sieve. The granules so produced are dried at 50°-60° C and passed through a No. 18 mesh U.S. sieve. The sodium carboxymethyl starch, magnesium stearate, and talc, previously passed through a No. 60 U.S. sieve, are then added to the granules which, after mixing, are compressed on a tablet machine to yield tablets.

Suspensions each containing 0.1 - 2000 mg of medicament per 5 mL dose are made as follows:

### Formulation 8: Suspensions

| Ingredient | Quantity (mg/5 ml) |
|---|---|
| Active ingredient | 0.1 - 2000 mg |
| Sodium carboxymethyl cellulose | 50 mg |
| Syrup | 1.25 mg |
| Benzoic acid solution | 0.10 mL |
| Flavor | q.v. |
| Color | q.v. |
| Purified water to | 5 mL |

The medicament is passed through a No. 45 mesh U.S. sieve and mixed with the sodium carboxymethyl cellulose and syrup to form a smooth paste. The benzoic acid solution, flavor, and color are diluted with some of the water and added, with stirring. Sufficient water is then added to produce the required volume.

### TEST PROCEDURE

The cells used to analyze the reversal of drug resistance are the human leukemia cell lines HL60/s, HL60/Vinc, and HL60/ADR. HL60/s cells are not selected for drug resistance and are therefore sensitive to drug treatment (McGrath, *et al.,* "Mechanisms of Multidrug Resistance in HL60 Cells. Analysis of Resistance Associated Membrane Proteins and Levels of *MDR* Gene Expression, *Biochemical Pharmacology, 38*: 3611-3619, 1989.). HL60/Vinc tumor cells are selected for resistance to vincristine and express a high level of the multidrug resistance-related protein, P-glycoProtein. *Id*. HL60/ADR cells are selected for resistance to Adriamycin but contain no detectable levels of P-glycoProtein. *Id*. The HL60/ADR cells, however, do contain a high amount of another resistance-related protein called P190 or MRP (Cole, *et al.,* "Overexpression of a Transporter Gene in a Multidrug-Resistant Human Lung Cancer Cell Line. *Science* (Washington DC), *258:* 1650-1654, 1992, Krishnamachary *et al.,* "The MRP Gene Associated with a Non-P-glycoProtein Multidrug Resistance Encodes a 190-kDa Membrane Bound Glycoprotein, *Cancer Research, 53*: 3658-3661, 1993.) The P-glycoProtein-containing cell line, HL60/Vinc, does not express P190.

The *in vitro* cytotoxicity assays are performed by suspending the target cells into Minimum Essential Eagle's Medium (MEM) containing 25% of the normal leucine concentration and 10% dialyzed fetal calf serum. 2x10⁴ cells are seeded into each well of 96 well microtiter plates and are incubated overnight in 5% CO₂ at 37°C. The agents to be tested for reversal activity are serially diluted (100 µM, 50 µM, ..., 0.2 µM) in media and added to the cells. 48 hours after adding the reversal agents to the target cells, 4 µCi of ³H-leucine are added to each well and are incubated for an additional 16 hours at 37°C. The cells are harvested and the amount of ³H-leucine incorporation is determined. The concentration of reversal agent required for 50% inhibition of ³H-leucine incorporation (IC₅₀) is calculated. Subsequent studies using reversal agents in the presence of cytotoxic drug (i.e., Adriamycin) utilize maximal concentrations of reversal agents that are not cytotoxic by themselves. The reversal of drug resistance by the various test compounds is accomplished by adding the maximum noncytotoxic concentration of the test compounds to log dilutions of Adriamycin (10, 1, 0.1, ... 0.001 µg/ml) and calculating the change in IC₅₀ observed for Adriamycin against the HL60/s, HL60/Vinc, and HL60/ADR cell lines in the above described *in vitro* cytotoxicity assay.

Activity in the above assay indicates the compounds, of the invention are potentially useful in treating resistant and succeptable neoplasms.

| | | Reversal Factor | |
|---|---|---|---|
| | Conc. µM | HL60/ADR | HL60/VINC |
| Compound A* | 12 | 5.7 | 3.8 |
| Raloxifene HCL | 6 | 7.1 | N.T. |
| 17∝ Estradiol | 3 | 1 | N.T. |
| Tamoxifen | 4 | 1.4 | 4.6 |

| | | | |
|---|---|---|---|
| *Compound A is of the formula I wherein R¹ and R³ are both hydrogen, and R² is 1-pyrrolidino. | | | |

## Claims

1. The use of a compound having the formula 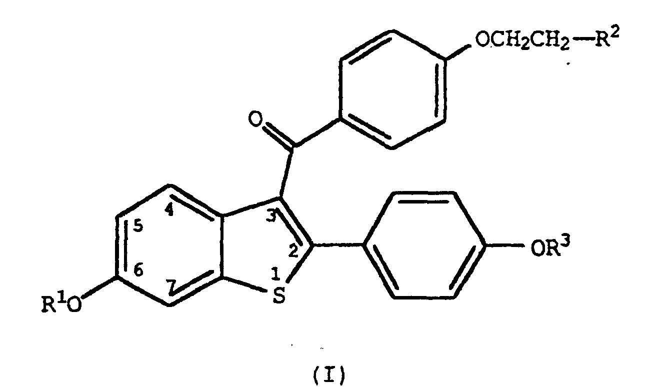 wherein R¹ and R³ are each hydrogen;
R² is piperidino; or a pharmaceutically acceptable salt or solvate thereof, in the preparation of a medicament for reversing drug resistance in a drug-resistant neoplasm.

2. The use of Claim 1 wherein said compound is the hydrochloride salt thereof.

3. The use of Claim 1 wherein said medicament is prophylactic.

## Patentansprüche

1. Verwendung einer Verbindung der Formel 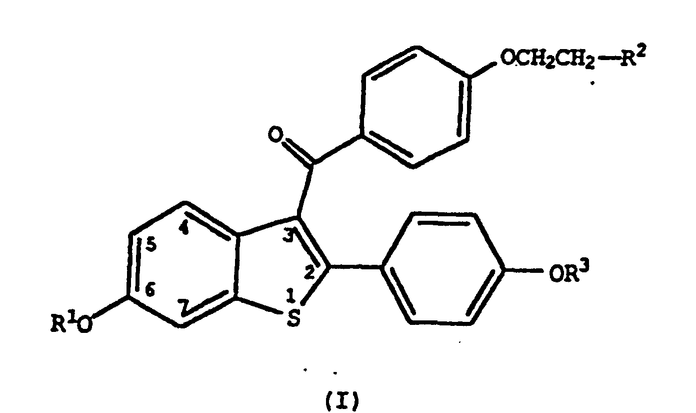 worin R¹ und R³ jeweils für Wasserstoff stehen,
R² für Piperidino steht,
oder eines pharmazeutisch annehmbaren Salzes oder Solvats hiervon zur Herstellung eines Arzneimittels zur Aufhebung der Arzneimittelresistenz bei einem arzneimittelresistenten Neoplasma.

2. Verwendung nach Anspruch 1, worin die Verbindung das Hydrochloridsalz hiervon ist.

3. Verwendung nach Anspruch 1, worin das Arzneimittel prophylaktisch ist.

## Revendications

1. Utilisation d'un composé répondant à la formule 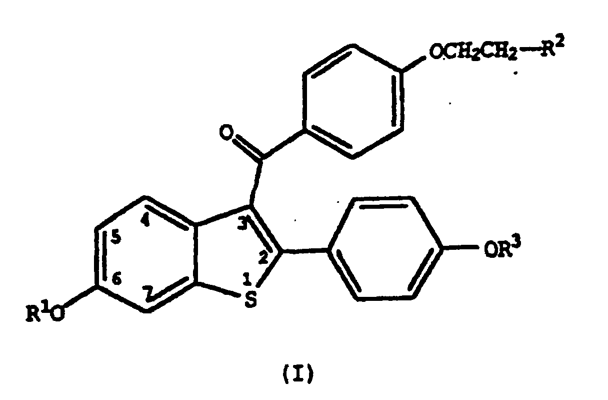 dans laquelle R¹ et R³ représentent chacun un atome d'hydrogène ;
R² représente un groupe pipéridino, ou d'un de ses sels ou de ses solvates pharmaceutiquement acceptables, dans la préparation d'un médicament pour contrecarrer la résistance à un médicament dans un néoplasme résistant à un médicament.

2. Utilisation selon la revendication 1, dans laquelle ledit composé est son sel chlorhydrate.

3. Utilisation selon la revendication 1, dans laquelle ledit médicament est un médicament prophylactique.
